Europäisches Patentamt

European Patent Office

Office européen des brevets

(11) Numéro de publication: **0 462 346 A1**

## (12) DEMANDE DE BREVET EUROPEEN

(21) Numéro de dépôt: 90403715.7

(22) Date de dépôt: 20.12.90

(51) Int. Cl.5: **E04F 17/12**

(30) Priorité: 20.06.90 FR 9007704

(43) Date de publication de la demande:
27.12.91 Bulletin 91/52

(84) Etats contractants désignés:
AT BE CH DE DK ES GB GR IT LI LU NL SE

(71) Demandeur: **Desgranges, Armand**
9 avenue Paul Doumer
F-91160 Saulx Les Chartreux(FR)

(72) Inventeur: **Desgranges, Armand**
9 avenue Paul Doumer
F-91160 Saulx Les Chartreux(FR)

(74) Mandataire: **Madeuf, Claude Alexandre Jean et al**
CABINET MADEUF 3, avenue Bugeaud
F-75116 Paris(FR)

(54) **Appareil permettant la désinfection et désodorisation en permanence des vide-ordures.**

(57) Appareil permettant en permanence la désinfection et la désodorisation d'un vide-ordures, cet appareil étant installé dans la zone de la colonne du vide-ordures placé au-dessus du réceptacle de déchets et d'ordures, caractérisé en ce qu'il comporte un réservoir de liquide désinfectant et désodorisant à partir duquel est prélevé, à travers un organe de réglage de débit (8) pouvant être muni d'un filtre, du liquide qui est amené par un conduit (14) à la tête de nébulisation (15) placé au centre d'un conduit d'air (11) alimenté en air pulsé par un moteur nébulisateur (9) prélevant l'air à projeter à travers un filtre (10), le moteur nébulisateur (9) étant soumis pour son alimentation électrique au contrôle d'un programmateur (22) muni d'un coupe-circuit (23), l'ensemble du dispositif étant maintenu sur un cadre support (2) comportant des pattes de fixation (1) rendant le cadre (2) solidaire, par exemple, d'un mur.

FIG. 2

FIG. 1

Dans les habitations modernes, la zone cuisine (cuisine ou séchoir à linge) comporte généralement un vide-ordures avec une pelle plus ou moins étanche. Du fait que les déchets et ordures ménagères tombant du vide-ordures dans une poubelle restent en moyenne 24 heures en bas du conduit, il se produit des dégagements donnant de mauvaises odeurs du fait également que le conduit est souillé en permanence par les ordures ou autres déchets. Il se produit donc un milieu de culture risquant de créer des émanations de composés infectieux nuisibles.

La loi oblige à la désinfection et à la désodorisation des vide-ordures en moyenne plusieurs fois par an, mais ces mesures ne sont pas suffisamment répétées pour assurer une parfaite désinfection et une parfaite désodorisation permanente du conduit.

La présente invention remédie à ces inconvénients en créant un appareil à commande automatique qui par nébulisation d'une solution de produits désinfectants et désodorisants, nettoie le conduit vertical et évite le développement de l'action microbiologique évitant la prolifération de germes biologiques et des odeurs nauséabondes.

L'appareil conforme à l'invention, de par sa conception et sa puissance, véhicule par projection une solution désinfectante et désodorisante sur toute la hauteur de la colonne du vide-ordures. Il est prévu également un réglage tel que la puissance de projection par nébulisation est suffisante pour que la ou les têtes de nébulisation ne soient pas obstruées par un dépôt éventuel d'ordures ou de déchets, ceux-ci étant chassés par le souffle de l'appareil. Finalement, il y a lieu d'indiquer qu'un cycle de désinfection ou de désodorisation est provoqué automatiquement pendant un court instant, de 1, 2 à 3 minutes en fonction de l'installation considérée à désinfecter et à désodoriser.

Conformément à l'invention, l'appareil permettant en permanence la désinfection et la désodorisation d'un vide-ordures, cet appareil étant installé dans la zone de la colonne du vide-ordures placée au-dessus du réceptacle de déchets et d'ordures, est caractérisé en ce qu'il comporte un réservoir de liquide désinfectant et désodorisant à partir duquel est prélevé, à travers un organe de réglage de débit pouvant être muni d'un filtre, du liquide qui est amené par un conduit à la tête de nébulisation placé au centre d'un conduit d'air alimenté en air pulsé par un moteur nébulisateur prélevant l'air à projeter à travers un filtre, le moteur nébulisateur étant soumis, pour son alimentation électrique, au contrôle d'un programmateur muni d'un coupe-circuit, l'ensemble du dispositif étant maintenu sur un cadre support comportant des pattes de fixation rendant le cadre solidaire, par exemple, d'un mur.

Diverses autres caractéristiques de l'invention ressortent d'ailleurs de la description détaillée qui suit.

Une forme de réalisation de l'objet de l'invention est représentée, à titre d'exemple non limitatif, aux dessins annexés.

La fig. 1 est une élévation de face schématique de l'appareil permettant la désinfection et la désodorisation en permanence des vide-ordures.

La fig. 2 est une vue de face d'un coffret de commande de l'appareil.

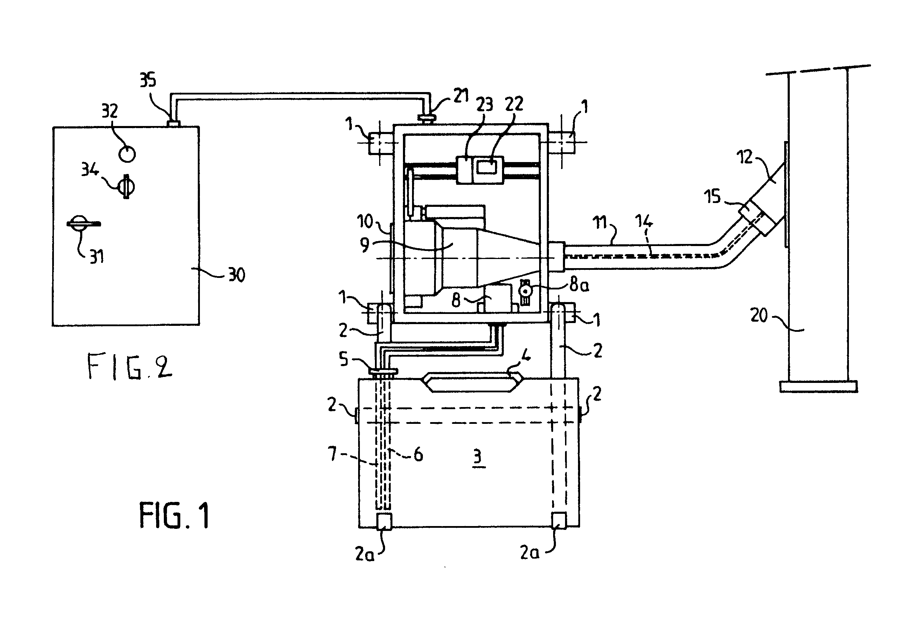

A la fig. 1, on a représenté schématiquement l'appareil qui se compose essentiellement de pattes de fixation 1 permettant d'accrocher l'appareil soit sur un mur, soit contre un mur. Les pattes inférieures 1 maintiennent un support 2 dont la base 2a formant équerre maintient un réservoir 3 de forme de parallélépipède rectangle réalisé le plus souvent en matière synthétique moulable présentant à sa partie supérieure une poignée de transport 4. Ce réservoir 3 présente, à sa face supérieure latérale, un conduit vertical 5 à travers lequel passent deux tubes 6, 7, l'un, le 6 est un tube d'aspiration et l'autre le 7, est un tube de refoulement. Ces tubes sont maintenus sur le conduit vertical 5 par un bouchon (non représenté) pouvant être engagé sur la face supérieure du conduit 5. Les tubes 6, 7 aboutissent à un organe 8 permettant le réglage du débit du liquide contenu dans le réservoir 3, ce liquide étant destiné à la désinfection et la désodorisation de la colonne 20 formant vide-ordures. Il est prévu sur l'organe 8 réglant le débit un filtre 8a. Un conduit, non représenté, partant de l'organe 8 aboutit à un groupe moteur nébulisateur 9 qui refoule ce liquide aspirant dans la colonne 20. Le moteur nébulisateur comporte un puissant ventilateur aspirant l'air à travers un filtre 10 pour le refouler dans un conduit d'air 11 aboutissant à un raccord platine de fixation 12 débouchant dans la colonne 20 formant vide-ordures (voir fig. 1). A la base de la colonne 20 est placé un récipient amovible destiné à recueillir les déchets et les ordures.

Le produit de désinfection et de désodorisation, qui est prélevé dans le réservoir 3 passe par le conduit d'aspiration 6 et l'organe 8 de réglage du débit et aboutit dans le conduit 14 concentrique au conduit d'air 11 et se termine dans une tête de nébulisation 15 placée à la partie inférieure du raccord platine de fixation 12. La dépression, créée par le passage de l'air pulsé dans le conduit 11, aspire le produit de désinfection et de désodorisation et le projette sous la forme d'un nuage de très fines gouttelettes dans la colonne formant vide-ordures 20 qui est soumise à une aspiration permanente vers le haut du fait que cette colonne débouche sur le toit du bâtiment et qu'ainsi se produit une aspiration permanente.

Comme cela est représenté à la fig. 1, le groupe moteur nébulisateur 9 est alimenté à partir de conducteurs électriques 21 à travers un programmateur 22 et un coupe-circuit général 23. Ainsi, ce groupe moteur nébulisateur 9 est protégé par le coupe-circuit 23 et est mis en marche et arrêté suivant les instructions données par le programmateur 22 qui a été programmé auparavent.

Il s'ensuit que lorsque l'appareil décrit et représenté à la fig. 1 est sous tension une projection de produit de désinfection et de désodorisation est projeté pendant un temps court par exemple 1, 2 et 3 minutes, toutes les heures ou plusieurs fois par jour dans la colonne 20 formant vide-ordures qui est aussi en permanence désinfectée et désodorisée.

La quantité du produit désinfectant et désodorisant est prélevée à chaque fois dans la réserve contenue dans le réservoir 3. La réserve est suffisante pour plusieurs semaines, voire plusieurs mois.

Par mesure de sécurité, il est prévu un coffret de commande 30 (voir fig. 2) qui est fermé à clef par un serrure 31 et qui comporte sur la face avant une lampetémoin 32 permettant de contrôler la mise sous tension de l'ensemble, puis en dessous un commutateur marche-arrêt 34.

35 désigne l'autre extrémité du conducteur d'alimentation 21 du groupe moteur nébulisateur 9.

Bien entendu, la puissance du groupe moteur nébulisateur est fonction du diamètre et de la hauteur de la colonne 20 et sa puissance évite dans tous les cas que la tête de nébulisation 15 s'obstrue par un dépôt éventuel d'ordures du fait que ces dernières sont chassées par le souffle de l'appareil. Dans certains cas, il est prévu plusieurs têtes de nébulisation 15 placées directement sur le même raccord 12 ou sur des raccords différents alimentés en air et en liquide par le même groupe moteur nébulisateur.

Cet appareil permet automatiquement une désinfection et une désodorisation de la colonne d'un vide-ordures avec une très large action microbiologique évitant ainsi la prolifération des germes pathogènes et des odeurs désagréables.

## Revendications

1. Appareil permettant en permanence la désinfection et la désodorisation d'un vide-ordures, cet appareil étant installé dans la zone de la colonne du vide-ordures placée au-dessus du réceptacle de déchets et d'ordures, caractérisé en ce qu'il comporte un réservoir (3) de liquide désinfectant et désodorisant à partir duquel est prélevé, à travers un organe de réglage de débit (8) pouvant être muni d'un filtre, du liquide qui est amené par un conduit (14) à la tête de nébulisation (15) placé au centre d'un conduit d'air (11) alimenté en air pulsé par un moteur nébulisateur (9) prélevant l'air à projeter à travers un filtre (10), le moteur nébulisateur (9) étant soumis, pour son alimentation électrique, au contrôle d'un programmateur (22) muni d'un coupe-circuit (23), l'ensemble du dispositif étant maintenu sur un cadre support (2) comportant des pattes de fixation (1) rendant le cadre (2) solidaire, par exemple, d'un mur.

2. Appareil suivant la revendication 1, caractérisé en ce que l'alimentation électrique de l'appareil est sous contrôle d'un coffret de commande hermétiquement fermé, comportant une lampe-témoin de tension (32) et un contacteur marche-arrêt (34).

3. Appareil suivant l'une des revendications 1 et 2, caractérisé en ce que le réservoir (3) contenant le liquide de désinfection et de désodorisation, qui est réalisé en matière plastique, est fixé sur la base du support (2).

4. Appareil suivant l'une des revendications 1 à 3, caractérisé en ce que le réservoir (3) comporte, à sa partie supérieure, un conduit vertical (5) muni de deux tubes (6, 7) d'aspiration et de refoulement du liquide, ces tubes étant maintenus sur la partie supérieure du conduit (5) par un bouchon.

5. Appareil suivant la revendication 1, caractérisé en ce qu'il peut comporter plusieurs têtes de nébulisation (15).

FIG.2

FIG.1

EP 0 462 346 A1

**Office européen
des brevets**

# RAPPORT DE RECHERCHE
# EUROPEENNE

Numéro de la demande

**EP 90 40 3715**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 000 003  (PETIT) <br> * page 2, line 6 - page 3, line 9; figure * <br> – – – | 1-3,5 | E 04 F 17/12 |
| A | FR-A-2 546 067  (SUBERVIELLE) <br> * page 1, line 8 - page 2, line 7; figure * <br> – – – | 1,3 | |
| A | FR-A-2 597 012  (TORTOCHOT) <br> * page 1, line 29 - page 3, line 4; figure * <br> – – – | 1-3 | |
| A | FR-A-2 600 896  (TAMAIN) <br> * page 4, line 15 - page 6, line 28; figure * <br> – – – – | 1,2,4,5 | |

**DOMAINES TECHNIQUES
RECHERCHES (Int. Cl.5)**

E 04 F
A 61 L

Le présent rapport de recherche a été établi pour toutes les revendications

| Lieu de la recherche | Date d'achèvement de la recherche | Examinateur |
|---|---|---|
| La Haye | 21 mars 91 | AYITER J. |

**CATEGORIE DES DOCUMENTS CITES**

X : particulièrement pertinent à lui seul
Y : particulièrement pertinent en combinaison avec un
autre document de la même catégorie
A : arrière-plan technologique
O : divulgation non-écrite
P : document intercalaire
T : théorie ou principe à la base de l'invention

E : document de brevet antérieur, mais publié à la
date de dépôt ou après cette date
D : cité dans la demande
L : cité pour d'autres raisons

&: membre de la même famille, document
correspondant